(19) Europäisches Patentamt
European Patent Office
Office européen des brevets

(11) Publication number: **0 509 518 A1**

# (12) EUROPEAN PATENT APPLICATION

(21) Application number: **92106647.8**

(51) Int. Cl.$^5$: **C07C 51/363**

(22) Date of filing: **16.04.92**

(30) Priority: **16.04.91 IT MI911052**

(43) Date of publication of application:
**21.10.92 Bulletin 92/43**

(84) Designated Contracting States:
**AT BE CH DE DK ES FR GB GR IT LI LU NL SE**

(71) Applicant: **ENICHEM AGRICOLTURA S.p.A.**
**Via Ruggero Settimo 55**
**I-90139 Palermo(IT)**

(72) Inventor: **Abbruzzese, Luigi**
**Via Pecorini, 14**
**I-20138 Milan(IT)**

(74) Representative: **von Hellfeld, Axel, Dr. Dipl.-Phys. et al**
**Wuesthoff & Wuesthoff Patent- und Rechtsanwälte Schweigerstrasse 2**
**W-8000 München 90(DE)**

## (54) Process for the preparation of 2,4-dichlorophenoxyacetic acid.

(57) A process is described for the preparation of 2,4-dichlorophenoxyacetic acid consisting of the chlorination of phenoxyacetic acid with chlorine, at temperatures from 0 °C to the boiling point of the reaction mixture, in a reaction medium composed of acetic acid.

EP 0 509 518 A1

The present invention relates to a process for the preparation of 2,4-dichlorophenoxyacetic acid which is completely free of dioxines.

2,4-dichlorophenoxyacetic acid is a well-known systematic weed-killer, commonly called 2,4-D and widely used also in the form of salts or esters for controlling weeds infesting cereals and other useful cultivations.

It is normally prepared, industrially, by a process which involves the chlorination of phenol in its molten state and subsequent condensation of the 2,4-dichlorophenol, thus obtained, with chloroacetic acid.

This process, however, has various draw-backs of which the most important is related to the fact that it does not guarantee the preparation of 2,4-D which is completely free of dioxines.

It is well-known that dioxines are highly toxic substances, even in very reduced quantities of parts per billion, both for human beings, animals and also plants.

In this respect the legislations of various countries are becoming more and more restrictive in establishing the quantities of dioxine which can be tolerated by operators exposed to this risk and which are acceptable for the safety of the environment.

The fact is that the above process, which is the most widely used in industrial production, even if excellent, always has the risk of the possible formation of undesired dioxines, both during the purification phase under basic conditions of the intermediate 2,4-dichlorophenol, and during its condensation with chloroacetic acid in a basic medium, bearing in mind that 2,4-dichlorophenol, even if previously purified, always contains traces of other chlorophenols precursors of polychlorodibenzodioxine.

Other disadvantages of the above process consist in the lengthy and costly purification operations which are necessary for obtaining a high quality product, together with the presence in the production plant of chlorophenols, extremely toxic and unpleasant-smelling substances which cause serious health problems for the operators and which require precise and costly techniques to avoid their negative impact on the surrounding environment.

Finally there are problems relating to the disposal of considerable quantities of bad-smelling waste products created by the condensation of the 2,4-dichlorophenol with chloroacetic acid.

A process has now been found for the preparation of 2,4-dichlorophenoxyacetic acid which, besides preventing the formation of dioxines, also overcomes the other draw-backs relating to the above-mentioned industrial process at present in use.

The present invention consequently relates to a process for the preparation of 2,4-dichlorophenoxyacetic acid consisting of chlorinating phenoxyacetic acid with chlorine, in a reaction medium composed of acetic acid, at temperatures ranging from 0 ° C to the boiling point of the reaction mixture.

The process may be schematically represented by the following equation:

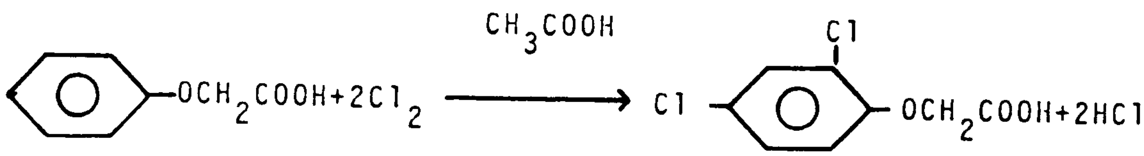


The quantities of acetic acid used in the process are not of paramount importance, however it is preferable, in order to reduce the production of waste to the minimum, to use weight ratios between the acetic acid and phenoxyacetic acid of between 1:2 and 1:3.

In practice it is preferable to operate at temperatures ranging from 50 ° C to 90 ° C.

Phenoxyacetic acid is a well-known product, available on the market, which can be prepared with known methods such as reacting phenol with chloroacetic acid in an alkaline environment in accordance with the following equation:

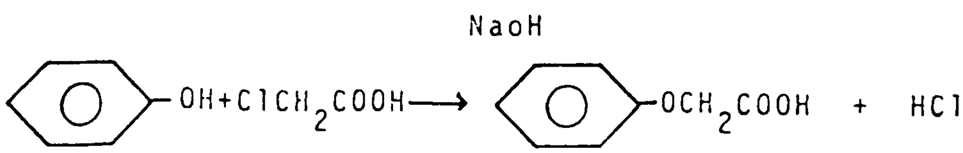


with the process of the present invention, a high quality 2,4-D is obtained, with a good yield and completely free of dioxines, in addition extremely limited quantities of waste are produced, which can be recycled without any specific draw-backs.

The following examples illustrate the present invention.

EXAMPLE 1

50 g of acetic acid are added to 30.4 g (0.1 moles) of phenoxyacetic acid and the mixture is heated to 50 ° C. At this temperature, chlorine is insufflated and the temperature is increased to 88 ° C over a period of 30 minutes.

The reaction is complete after 3 hours at this temperature.

The mixture is cooled under stirring to 15 ° C. A crystalline solid precipitates which is filtered, washed with a small amount of acetic acid and a small quantity of water, and is then left to dry. 33.7 g of 2,4-D are obtained, as a white crystalline solid, which melts at 135-138 ° C, with a purity of 98-100% and which, on analysis, is free of polychlorodibenzodioxines, such as for example, tetrachlorodioxines, such as 2,3,7,8-tetrachlorodibenzodioxine and pentachlorodioxines, within the limits of detection <0.1 micrograms/kg, and also free of hexachloro-, heptachloro- and octachloro-dioxines.

EXAMPLE 2

60 g of acetic acid are added to 30.4 g (0.2 moles) of phenoxyacetic acid, and the mixture is heated to 90 ° C. At this temperature, chlorine is insufflated.

Owing to the isothermal conditions, the temperature increases to 105 ° C. This temperature is maintained for 3 hours, after which the mixture is cooled under stirring to 10 ° C. A crystalline solid precipitates which is filtered, washed with a small amount of acid and water and is then left to dry.

34.7 g of 2,4-D are obtained as a white crystalline solid, which melts at 134-136 ° C and has a purity of 93-95%, and which, similar to the product of Example 1, upon analysis is completely free of polychlorodibenzodioxines.

**Claims**

1. Process for the preparation of 2,4-dichlorophenoxyacetic acid consisting of chlorinating phenoxyacetic with chlorine, in a reaction medium composed of acetic acid, at temperatures ranging from 0 ° C to the boiling point of the reaction mixture.

2. Process in accordance with Claim 1, wherein the chlorination is carried out at temperatures ranging from 50 ° C to 90 ° C, with a weight ratio between the acetic acid and phenoxyacetic acid of between 1:2 and 1:3.

European Patent Office

# EUROPEAN SEARCH REPORT

Application Number

EP 92 10 6647

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | GB-A-607 113 (BOOTS PURE DRUG CO.)<br>* page 2, line 67 - line 95 *<br>* page 3; example 2 *<br>* claims 1-3,5-8 *<br>--- | 1,2 | C07C51/363 |
| Y | GB-A-627 709 (LEON HASKELBERG)<br>* page 2; example 1 *<br>* claim 1 *<br>--- | 1 | |
| Y | US-A-2 774 788 (A.HLYNSKY ET AL.)<br>* column 2, line 23 - line 29; claim 1 *<br>----- | 1 | |
| | | | **TECHNICAL FIELDS SEARCHED (Int. Cl.5 )** |
| | | | C07C |

The present search report has been drawn up for all claims

| Place of search | Date of completion of the search | Examiner |
|---|---|---|
| THE HAGUE | 17 JUNE 1992 | KLAG M.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)